# EUROPÄISCHE PATENTSCHRIFT

(11) **EP 1 398 075 B1**
(45) Veröffentlichungstag und Bekanntmachung des Hinweises auf die Patenterteilung: **07.03.2007**
(21) Anmeldenummer: 02020212.3
(22) Anmeldetag: 10.09.2002
(51) Int. Cl.: B01J 13/16, A61K 8/11, D06M 23/12

(54) **Polyamidmikrokapseln (IV)**
Polyamide microcapsules (IV)
Microcapsules de polyamide (IV)

(43) Veröffentlichungstag der Anmeldung: 17.03.2004
(73) Patentinhaber: Cognis IP Management GmbH, 40589 Düsseldorf (DE)
(72) Erfinder: Viladot Petit, Josep-Lluis, Dr., 08018 Barcelona (ES); Tacies, Anna, 08034 Barcelona (ES)

(56) Entgegenhaltungen:
- GB-A- 1 551 142
- US-A- 4 447 516

## Beschreibung

### Gebiet der Erfindung

Die Erfindung befindet sich auf dem Gebiet der verkapselten Wirkstoffe und betrifft neue Mikrokapseln mit Polyamidhüllen, ein Verfahren zu deren Herstellung sowie deren Verwendung in einer Reihe von Einsatzgebieten.

### Stand der Technik

Unter dem Begriff "Mikrokapsel" werden vom Fachmann sphärische Aggregate mit einem Durchmesser im Bereich von etwa 0,0001 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Die mikroskopisch kleinen Kapseln, auch Nanokapseln genannt, lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hülhnaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) *: Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und Primasys (Phospholipide).

Chitosanmikrokapseln und Verfahren zu ihrer Herstellung sind Gegenstand früherer Patenanmeldungen der Patentanmelderin **[WO 01/01926, WO 01/01927, WO 01/01928, WO 01/01929].** Mikrokapseln mit mittleren Durchmessern im Bereich von 0,0001 bis 5, vorzugsweise 0,001 bis 0,5 und insbesondere 0,005 bis 0,1 mm, bestehend aus einer Hüllmembran und einer die Wirkstoffe enthaltenden Matrix, können beispielsweise erhalten werden, indem man
(a1) aus Gelbildnern, Chitosanen und Wirkstoffen eine Matrix zubereitet,
(a2) gegebenenfalls die Matrix in einer Ölphase dispergiert,
(a3) die dispergierte Matrix mit wässrigen Lösungen anionischer Polymere behandelt und gegebenenfalls dabei die Ölphase entfernt.
oderµ
(b1) aus Gelbildnern, anionischen Polymeren und Wirkstoffen eine Matrix zubereitet,
(b2) gegebenenfalls die Matrix in einer Ölphase dispergiert,
(b3) die dispergierte Matrix mit wässrigen Chitosanlösungen behandelt und gegebenenfalls dabei die Ölphase entfernt.
oder
(c1) wässrige Wirkstoffzubereitungen mit Ölkörpern in Gegenwart von Emulgatoren zu O/W-Emulsionen verarbeitet,
(c2) die so erhaltenen Emulsionen mit wässrigen Lösungen anionischer Polymere behandelt,
(c3) die so erhaltene Matrix mit wässrigen Chitosanlösungen in Kontakt bringt und
(c4) die so erhaltenen Verkapselungsprodukte von der wässrigen Phase abtrennt.

Von Nachteil ist jedoch, dass die Mikrokapseln des Stands der Technik gegenüber aggressiven Medien nur sehr bedingt stabil sind. Kapseln des oben genannten Typs lösen sich beispielsweise in Hypochloritlösungen oder Wasserstoffperoxid schlicht auf, so dass sie mit diesen Stoffen nicht in Kontakt gebracht werden können. Andererseits ist es gerade für den amerikanischen und den südeuropäischen Raum, in dem Einsatz von solchen Mitteln für die Reinigung harter Oberflächen sehr verbreitet ist, wünschenswert, Mikrokapseln zur Verfügung stellen zu können, die sich in diesen Medien als hinreichend stabil erweisen und die darin enthaltenen Wirkstoffe erst nach Zuführung mechanischer Energie freisetzen.

Die Aufgabe der vorliegenden Erfindung hat folglich darin bestanden, neue Mikrokapseln mit mittleren Durchmessern im sichtbaren Bereich von 0,1 bis 5 mm zur Verfügung zu stellen, die in hypochlorit- bzw. wasserstoffperoxidhaltigen Lösungen stabil sind, d.h. sich über einen Zeitraum von wenigstens 4 Wochen nicht auflösen oder den sie enthaltenen Wirkstoff anders freisetzen.

### Beschreibung der Erfindung

Gegenstand der Erfindung sind neue Polyamidmikrokapseln mit einem mittleren Durchmesser im Bereich von 0,1 bis 5 mm, die dadurch erhältlich sind, dass man
(a) eine wässrige Phase herstellt, in der (a1) Diamine und/oder Triamine und (a2) Tenside gelöst sind,
(b) eine Ölphase herstellt, in der (b1) Dicarbonsäurechloride, (b2) Wachskörper sowie gegebenenfalls (b3) Wirkstoffe gelöst sind, und
(c) die beiden Phasen unter Ausbildung einer O/W-Emulsion in Kontakt bringt, so dass an den Grenzflächen eine Polykondensationsreaktion unter Bildung von Polyamidmikrokapseln stattfindet.

Überraschenderweise wurde gefunden, dass sich gerade Polyamidkapseln durch eine besondere Beständigkeit in Hypochloritlaugen und peroxidischen Mitteln auszeichnen, wozu es jedoch erforderlich ist, nach der Grenzflächenpolykondensation eine Aushärtung, d.h. Ausbildung von Verzweigungen in der Kapselhülle durch Calciumionen durchzuführen. Die so erhaltenen sphärischen Gebilde haben einen mittleren Durchmesser von wenigstens 0,1 mm und sind über einen Zeitraum von wenigstens 4 Wochen in gängigen hypochlorithaltigen Haushaltsreinigem beständig.

### Verfahren

Ein weiterer Gegenstand der Erfindung betrifft ein Verfahren zur Herstellung von Polyamidmikrokapseln mit einem mittleren Durchmesser im Bereich von 0,1 bis 5 mm, bei dem man
(a) eine wässrige Phase herstellt, in der (a1) Diamine und/oder Triamine und (a2) Tenside gelöst sind,
(b) eine Ölphase herstellt, in der (b1) Dicarbonsäurechloride und (b2) Wachskörper sowie gegebenenfalls (b3) Wirkstoffe gelöst sind, und
(c) die beiden Phasen unter Ausbildung einer O/W-Emulsion in Kontakt bringt, so dass an den Grenzflächen eine Polykondensationsreaktion unter Bildung von Polyamidmikrokapseln stattfindet.

### Diamine und Triamine

In einem ersten Schritt des erfindungsgemäßen Verfahrens wird eine wässrige Zubereitung hergestellt, welche als Komponente (a1) das für die Polykondensation benötigte Di- und/oder Triamin enthält. Die Auswahl dieser Komponente ist wenig kritisch, demnach kommen grundsätzlich alle aliphatischen oder aromatischen Di- oder Triamine in Betracht, die über eine hinreichende Wasserlöslichkeit verfügen. Besonders bevorzugt ist indes das für Nylon-6,6 bekannte Hexamethylendiamin. Der Einsatz von Triaminen bietet infolge der bei der Polykondensation stattfindenden Vernetzung den Vorteil, dass die Mikrokapseln stabiler werden. Die wässrige Phase enthält die Komponente (a1) üblicherweise in Mengen von 0,1 bis 5, vorzugsweise 0,5 bis 2 Gew.-%.

### Tenside

Als Komponente (a2) kommen sowohl anionische als auch kationische, amphotere oder zwitterionische oberflächenaktive, zur - O/W - Emulsionsbildung befähigte Verbindungen in Betracht. Vorzugsweise werden jedoch nichtionische Tenside eingesetzt. Typische Beispiele für nichtionische Tenside bzw. Emulgatoren (Komponente a2) sind
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢Glycerincarbonat.

### ➢Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

### ➢ Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

### ➢ Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

### ➢ Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

### ➢ Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Die wässrige Phase enthält die Komponente (a2) üblicherweise in Mengen von 0,1 bis 5, vorzugsweise 0,5 bis 2 Gew.-%.

### Ölphase

Im zweiten Verfahrensschritt wird die Ölphase hergestellt, welche die zweite Kondensationskomponente, nämlich das Dicarbonsäurechlorid (Komponente b1), Wachskörper (Komponente b2) sowie gegebenenfalls die Wirkstoffe (Komponente b3) enthält. Auch hier ist die Auswahl der Dicarbonsäurekomponente wenig kritisch, im Hinblick auf die Herstellung von Nylon-6,6-Kapseln empfiehlt sich natürlich wieder Sebacinsäurechlorid. Alternativ können aber auch längerkettige Dicarbonsäuren, wie die bei der Fermentation von entsprechenden Paraffinen anfallende 1,18-Hexadecandicarbonsäure bzw. deren Chlorid eingesetzt werden. Auch die Auswahl des Öls ist wenig kritisch. In der Regel wird man Mineralöl ("Weißöl") bzw. Paraffinöl einsetzen, ebenfalls geeignet sind auch Siliconöle oder Esteröle sowie alle übrigen gängigen kosmetischen Ölkörper. Die Ölphase enthält die Komponente (b1) üblicherweise in Mengen von 0,1 bis 5 und vorzugsweise 2 bis 3 Gew.-%.

### Wachskörper

Typische Beispiele Wachskörper (Komponente b2) sind Fette bzw. Glyceride, d.h. bei Raumtemperatur flüssige, vorzugsweise aber feste pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage. Die Ölphase enthält die Komponente (b2) üblicherweise in Mengen von 0,1 bis 5 und vorzugsweise 2 bis 3 Gew.-%.

### Wirkstoffe

In einer bevorzugten Ausführungsform der vorliegenden Erfindung enthalten die Mikrokapseln in ihrer inneren ölhaltigen Phase - vorzugsweise öllösliche bzw. öldispergierbare - Wirkstoffe (Komponente b3), deren Auswahl weitgehend unkritisch ist und sich ausschließlich nach der Aufgabenstellung richtet. Typische Beispiele sind Enzyme, biogene Wirkstoffe, Antioxidantien, UV-Filter, Parfümöle und Aromen, Farbstoffe bzw. Farbpigmente sowie vollständige kosmetische oder pharmazeutische Emulsionen. Im Hinblick auf Verwendungen in Gegenwart von Hypochloriten oder Peroxiden ist der Einsatz von Enzymen, Farb- und Duftstoffen bevorzugt, die ansonsten in dieser Umgebung chemisch instabil wären. Nichtsdestotrotz können die Kapseln natürlich auch in wässriger Umgebung, also beispielsweise in der Kosmetik oder der Textiltechnik eingesetzt werden. Die Ölphase enthält die Komponente (b3) üblicherweise in Mengen von 0,1 bis 25 und vorzugsweise 2 bis 3 Gew.-%.

### ➢ Enzyme

Als Enzyme kommen insbesondere solche aus der Klasse der Hydrolasen, wie der Proteasen, Esterasen, Lipasen bzw. lipolytisch wirkenden Enzyme, Amylasen, Cellulasen bzw. andere Glykosylhydrolasen und Gemische der genannten Enzyme in Frage. Alle diese Hydrolasen tragen in der Wäsche zur Entfernung von Verfleckungen, wie protein-, fettoder stärkehaltigen Verfleckungen, und Vergrauungen bei. Cellulasen und andere Glykosylhydrolasen können durch das Entfernen von Pilling und Mikrofibrillen zur Farberhaltung und zur Erhöhung der Weichheit des Textils beitragen. Zur Bleiche bzw. zur Hemmung der Farbübertragung können auch Oxidoreduktasen eingesetzt werden. Besonders gut geeignet sind aus Bakterienstämmen oder Pilzen, wie Bacillus subtilis, Bacillus licheniformis, Streptomyces griseus und Humicola insolens gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease- und/oder Lipase-haltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder O-xidasen haben sich in einigen Fällen als geeignet erwiesen. Zu den geeigneten Amylasen zählen insbesondere α-Amylasen, Iso-Amylasen, Pullulanasen und Pektinasen. Als Cellulasen werden vorzugsweise Cellobiohydrolasen, Endoglucanasen und β-Glucosidasen, die auch Cellobiasen genannt werden, bzw. Mischungen aus diesen eingesetzt. Da sich die verschiedenen Cellulase-Typen durch ihre CMCase- und Avicelase-Aktivitäten unterscheiden, können durch gezielte Mischungen der Cellulasen die gewünschten Aktivitäten eingestellt werden.

### ➢ Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Dihydroxyaceton, Arbutin, Ferulasäure, Kojisäure, Cumarinsäure und Ascorbinsäure (Vitamin C), Aminosäuren, Ceramide, Pseudoceramide, essentielle Öle, Pflanzenextrakte, wie z.B. Prunusextrakt, Bambaranussextrakt und Vitaminkomplexe zu verstehen.

### ➢ Antioxidantien

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesterylund Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, U-bichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### ➢ UV-Lichtschutzfilter

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher beschrieben;
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)-benzoesäureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4' -methylbenzophenon, 2,2' -Dihydroxy-4-methoxybenzophenon;
➢ Ester der Benzahnalonsäure, vorzugsweise 4-Methoxybenzmalonsäuredi-2-ethylhexylester;
➢ Triazinderivate, wie z.B. 2,4,6-Trianilino-(p-carbo-2`-ethyl-1'-hexyloxy)-1,3,5-triazin und Octyl Triazon oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzo-phenon-5-sulfonsäure und ihre Salze;
➢ Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4`-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

### ➢ Antischuppenwirkstoffe

Typische Beispiele für Antischuppenwirkstoffe sind Pirocton, Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl)r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfelteer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat.

### ➢ Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, I-raldein gamma, Phenylessigsäure, Geranylacetat, Benzylacetat, Rosenoxid, Romilllat, I-rotyl und Floramat allein oder in Mischungen, eingesetzt. Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### ➢ Farbstoffe und Farbpigmente

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984**, **S.81-106** zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein.

### Emulsionsbildung, Polykondensation, Härtung und Aufarbeitung

Im dritten Schritt findet die eigentliche Phasengrenzflächenpolykondensation statt. Hierzu werden die wässrige Phase und die Ölphase unter starker Scherung mit einander vermischt, wobei eine O/W-Emulsion erhalten wird. Typischerweise setzt man die beiden Phasen im Gewichtsverhältnis 3 : 1 bis 1 : 3 und vorzugsweise etwa 2 : 1 bis 1 : 2 ein. An den Grenzflächen der Öltröpfchen zu der sie umgebenden Wasserphase findet eine Polykondensation zwischen den Diaminen und den Dicarbonsäurechloriden statt und es bilden sich sphärische Gebilde mit einer Polyamidhülle und einer öligen inneren Phase, in der die Wirkstoffe enthalten sind. Anschließend werden die Lösungen abgekühlt, so dass die Wachskörper erstarren. Diese Mikrokapseln werden vorsichtig abfiltriert und mit Wasser oder verdünnter Tensidlösung gewaschen, um anhaftende Reste der Ölphase zu entfernen.

### Gewerbliche Anwendbarkeit

Ein weiterer Gegenstand der Erfindung betrifft die Verwendung der neuen Polyamidmikrokapseln zur Herstellung von hypochlorithaltigen Zubereitungen, speziell von Mitteln zur Reinigung harter Oberflächen, welche die Kapseln in Mengen von 1 bis 10 Gew.-% - bezogen auf die Mittel - enthalten können. Gleichwohl eignen sich die Mikrokapseln auch für andere Anwendungszwecke, beispielsweise zur Herstellung von kosmetischen Zubereitungen und zur Ausrüstung von textilen Flächengebilden.

### Beispiele

### Beispiel 1

In einem 1-1-Dreihalskolben mit Rührer und Tropftrichter wurden bei Raumtemperatur 0,3 g Hexamethylendiamin, 0,5 g Sorbitanmonolaurat+20EO und 0,05 g Phenonip vorgelegt und mit destilliertem Wasser auf 100 ml aufgefüllt. Zu der Lösung wurde unter starkem Rühren innerhalb von 3 min 100 g einer 5 Gew.-%igen Lösung von Sebacinsäurechlorid in Paraffinöl, die zudem noch 0,1 g Paraffinwachs und 0,05 g Indanthrenblau RS (C.I. 69800) enthielt, getropft. Nach etwa 30 min war die Polykondensation abgeschlossen. Die Lösungen wurden abgekühlt, bis der Wachskörper sich verfestigt hatte, die erhaltenen Polyamidmikrokapseln vorsichtig über einem Büchnerfilter von der Ölphase abgetrennt und mit einer 3 Gew.-% Natriumlaurylsulfatlösung gewaschen, bis keine Ölreste mehr zu erkennen waren. Der mittlere Durchmesser der Kapseln betrug 1 mm.

### Beispiel 2

In einem 1-1-Dreihalskolben mit Rührer und Tropftrichter wurden bei Raumtemperatur 0,3 g Hexamethylendiamin, 0,5 g einer 1:1 Mischung aus Cocoglucosides und Polyglyceryl-2 Dipolyhydroxystearat, und 0,05 g Phenonip vorgelegt und mit destilliertem Wasser auf 100 ml aufgefüllt. Zu der Lösung wurde unter starkem Rühren innerhalb von 3 min 100 g einer 5 Gew.-%igen Lösung von Sebacinsäurechlorid in Paraffinöl, die zudem noch 0,2 g Cera Alba und 0,05 g Krapplack (C.I.58000) enthielt, getropft. Nach etwa 30 min war die Polykondensation abgeschlossen. Die Lösungen wurden abgekühlt, bis der Wachskörper sich verfestigt hatte, die erhaltenen Polyamidmikrokapseln vorsichtig über einem Büchnerfilter von der Ölphase abgetrennt und mit einer 3 Gew.-% Natriumlaurylsulfatlösung gewaschen, bis keine Ölreste mehr zu erkennen waren. Der mittlere Durchmesser der Kapseln betrug 1 mm.

### Beispiel 3

In einem 1-1-Dreihalskolben mit Rührer und Tropftrichter wurden bei Raumtemperatur 0,3 g Hexamethylendiamin, 0,5 g Sorbitansesquioleat+20EO und 0,05 g Phenonip vorgelegt und mit destilliertem Wasser auf 100 ml aufgefüllt. Zu der Lösung wurde unter starkem Rühren innerhalb von 3 min 100 g einer 3 Gew.-%igen Lösung von 1,18-Hexadecandicarbonsäurechlorid in Paraffinöl, die zudem noch 0,1 g Polyolefinwachs und 0,1 g Retinol enthielt, getropft. Nach etwa 30 min war die Polykondensation abgeschlossen. Die Lösungen wurden abgekühlt, bis der Wachskörper sich verfestigt hatte, die erhaltenen Polyamidmikrokapseln vorsichtig über einem Büchnerfilter von der Ölphase abgetrennt und mit einer 3 Gew.-% Natriumlaurylsulfatlösung gewaschen, bis keine Ölreste mehr zu erkennen waren. Der mittlere Durchmesser der Kapseln betrug 1 mm.

### Beispiel 4

Die gemäß Beispiele 1 bis 3 erhaltenen Polyamidmikrokapseln wurden in eine 5 Gew.-%ige Natriumhydroxidlösung eingebracht und dort über einen Zeitraum von 4 Wochen bei 30 °C gelagert. Alle Systeme erwiesen sich dabei als stabil.

## Patentansprüche

1. Polyamidmikrokapseln mit einem mittleren Durchmesser im Bereich von 0,1 bis 5 mm, dadurch erhältlich, dass man
(a) eine wässrige Phase herstellt, in der (a1) Diamine und/oder Triamine und (a2) Tenside gelöst sind,
(b) eine Ölphase herstellt, in der (b1) Dicarbonsäurechloride, (b2) Wachskörper und gegebenenfalls (b3) Wirkstoffe gelöst sind, und
(c) die beiden Phasen unter Ausbildung einer O/W-Emulsion in Kontakt bringt, so dass an den Grenzflächen eine Polykondensationsreaktion unter Bildung von Polyamidmikrokapseln stattfindet.

2. Verfahren zur Herstellung von Polyamidmikrokapseln mit einem mittleren Durchmesser im Bereich von 0,1 bis 5 mm, bei dem man
(a) eine wässrige Phase herstellt, in der (a1) Diamine und/oder Triamine und (a2) Tenside gelöst sind,
(b) eine Ölphase herstellt, in der (b1) Dicarbonsäurechloride, (b2) Wachskörper und gegebenenfalls (b3) Wirkstoffe gelöst sind, und
(c) die beiden Phasen unter Ausbildung einer O/W-Emulsion in Kontakt bringt, so dass an den Grenzflächen eine Polykondensationsreaktion unter Bildung von Polyamidmikrokapseln stattfindet.

3. Verfahren nach Anspruch 2, **dadurch gekennzeichnet, dass** man als Diamin (Komponente a1) Hexamethylendiamin einsetzt.

4. Verfahren nach den Ansprüchen 2 und/oder 3, **dadurch gekennzeichnet, dass** man als Komponente (a2) nichtionische Tenside einsetzt, die ausgewählt sind aus der Gruppe, die gebildet wird von
(a2-1) Anlagerungsprodukten von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest,
(a2-2) Alkyl- und/oder Alkenyloligoglykosiden mit 8 bis 22 Kohlenstoffatomen im Alk(en)ylrest und deren ethoxylierten Analoga,
(a2-3) Anlagerungsprodukten von 1 bis 15 Mol Ethylenoxid an Ricinusöl und/oder gehärtetes Ricinusöl,
(a2-4) Anlagerungsprodukten von 15 bis 60 Mol Ethylenoxid an Ricinusöl und/oder gehärtetes Ricinusöl,
(a2-5) Partialestern von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukten mit 1 bis 30 Mol Ethylenoxid,
(a2-6) Partialestern von Polyglycerin, Polyethylenglycol, Trimethylolpropan, Pentaerythrit, Zuckeralkoholen, Alkylglucosiden sowie Polyglucosiden mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukten mit 1 bis 30 Mol Ethylenoxid,
(a2-7) Mischestern aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin,
(a2-8) Mono-, Di- und Trialkylphosphaten sowie Mono-, Di- und/oder Tri-PEGalkylphosphaten und deren Salzen,
(a2-9) Wollwachsalkoholen,
(a2-10) Polysiloxan-Polyalkyl-Polyether-Copolymeren bzw. entsprechenden Derivaten,
(a2-11) Block-Copolymeren, Polymeremulgatoren, Polyalkylenglycolen sowie
(a2-12) Glycerincarbonat.

5. Verfahren nach mindestens einem der Ansprüche 2 bis 4, **dadurch gekennzeichnet, dass** man wässrige Phasen einsetzt, die die Komponenten (a1) und (a2) in Mengen von jeweils 0,1 bis 5 Gew.-% enthalten.

6. Verfahren nach mindestens einem der Ansprüche 2 bis 5, **dadurch gekennzeichnet, dass** man als Komponente (b1) Sebacinsäurechlorid einsetzt.

7. Verfahren nach mindestens einem der Ansprüche 2 bis 6, **dadurch gekennzeichnet, dass** man als Komponente (b2) Wachskörper einsetzt, die ausgewählt sind aus der Gruppe, die gebildet wird von natürlichen Wachsen, Petrolatum, Paraffinwachsen, Mikrowachsen, Hartwachsen, Polyalkylenwachsen, Polyolefinwachsen, Lecithinen und Phospholipiden.

8. Verfahren nach mindestens einem der Ansprüche 2 bis 7, **dadurch gekennzeichnet, dass** man Ölphasen einsetzt, die die Komponenten (b1) und (b2) in Mengen von jeweils 0,1 bis 5 Gew.-% enthalten.

9. Verfahren nach mindestens einem der Ansprüche 2 bis 8, **dadurch gekennzeichnet, dass** man als Komponente (b3) Wirkstoffe einsetzt, die ausgewählt sind aus der Gruppe, die gebildet wird von Enzymen, biogenen Wirkstoffen, Antioxidantien, UV-Filtem, Parfümölen und Aromen, Farbstoffen bzw. Farbpigmenten sowie vollständige kosmetischen oder pharmazeutischen Emulsionen.

10. Verfahren nach mindestens einem der Ansprüche 2 bis 9, **dadurch gekennzeichnet, dass** man Ölphasen einsetzt, die die Komponente (b3) in Mengen von 0,1 bis 25 Gew.-% enthalten.

11. Verfahren nach mindestens einem der Ansprüche 2 bis 10, **dadurch gekennzeichnet, dass** man die wässrige Phase und die Ölphase im Gewichtsverhältnis 3 : 1 bis 1 : 3 einsetzt.

12. Verwendung von Polyamidmikrokapseln nach Anspruch 1 zur Herstellung von hypochlorithaltigen Zubereitungen.

13. Verwendung von Polyamidmikrokapseln nach Anspruch 1 zur Herstellung von kosmetischen Zubereitungen.

14. Verwendung von Polyamidmikrokapseln nach Anspruch 1 zur Ausrüstung von textilen Flächengebilden.

## Claims

1. Polyamide microcapsules with a mean diameter of 0.1 to 5 mm obtainable by
(a) preparing an aqueous phase in which (a1) diamines and/or triamines and (a2) surfactants are dissolved,
(b) preparing an oil phase in which (b1) dicarboxylic acid chlorides, (b2) waxes and optionally (b3) active components are dissolved,
(c) contacting the two phases to form a w/o emulsion, so that a polycondensation reaction takes place at the interfaces, resulting in the formation of polyamide microcapsules.

2. A process for the production of polyamide microcapsules with a mean diameter of 0.1 to 5 mm comprising the steps of
(a) preparing an aqueous phase in which (a1) diamines and/or triamines and (a2) surfactants are dissolved,
(b) preparing an oil phase in which (b1) dicarboxylic acid chlorides, (b2) waxes and optionally (b3) active components are dissolved,
(c) contacting the two phases to form a w/o emulsion, so that a polycondensation reaction takes place at the interfaces, resulting in the formation of polyamide microcapsules.

3. A process as claimed in claim 2, **characterized in that** hexamethylenediamine is used as the diamine (component a1).

4. A process as claimed in claims 2 and/or 3, **characterized in that** nonionic surfactants selected from the group consisting of
(a2-1) products of the addition of 2 to 30 mol ethylene oxide and/or 0 to 5 mol propylene oxide onto linear C₈₋₂₂ fatty alcohols, onto C₁₂₋₂₂ fatty acids, onto alkyl phenols containing 8 to 15 carbon atoms in the alkyl group and alkylamines containing 8 to 22 carbon atoms in the alkyl group;
(a2-2) alkyl and/or alkenyl oligoglycosides containing 8 to 22 carbon atoms in the alkyl group and ethoxylated analogs thereof;
(a2-3) addition products of 1 to 15 mol ethylene oxide onto castor oil and/or hydrogenated castor oil;
(a2-4) addition products of 15 to 60 mol ethylene oxide onto castor oil and/or hydrogenated castor oil;
(a2-5) partial esters of glycerol and/or sorbitan sorbitan with unsaturated, linear or saturated, branched fatty acids containing 12 to 22 carbon atoms and/or hydroxycarboxylic acids containing 3 to 18 carbon atoms and adducts thereof with 1 to 30 mol ethylene oxide;
(a2-6) partial esters of polyglycerol, polyethylene glycol, trimethylolpropane, pentaerythritol, sugar alcohols, alkyl glucosides and polyglucosides with saturated and/or unsaturated, linear or branched fatty acids containing 12 to 22 carbon atoms and/or hydroxycarboxylic acids containing 3 to 18 carbon atoms and adducts thereof with 1 to 30 mol ethylene oxide;
(a2-7) mixed esters of pentaerythritol, fatty acids, citric acid and fatty alcohol and/or mixed esters of fatty acids containing 6 to 22 carbon atoms, methyl glucose and polyols, preferably glycerol or polyglycerol,
(a2-8) mono-, di- and trialkyl phosphates and mono-, di- and/or tri-PEG-alkyl phosphates and salts thereof,
(a2-9) wool wax alcohols,
(a2-10) polysiloxane/polyalkyl/polyether copolymers and corresponding derivatives,
(a2-11) block copolymers, polymer emulsifiers, polyalkylene glycols and
(a2-12) glycerol carbonate
are used as component (a2).

5. A process as claimed in at least one of claims 2 to 4, **characterized in that** aqueous phases containing components (a1) and (a2) and (a3) in quantities of 0.1 to 5% by weight each are used.

6. A process as claimed in at least one of claims 2 to 5, **characterized in that** sebacic acid chloride is used as component (b1).

7. A process as claimed in at least one of claims 2 to 6, **characterized in that** waxes selected from the group consisting of natural waxes, petrolatum, paraffin waxes, microwaxes, hard waxes, polyalkylene waxes, polyolefin waxes, lecithins and phospholipids are used as component (b2).

8. A process as claimed in at least one of claims 2 to 7, **characterized in that** oil phases containing components (b1) and (b2) in quantities of 0.1 to 5% by weight each are used.

9. A process as claimed in at least one of claims 2 to 8, **characterized in that** active components selected from the group consisting of enzymes, biogenic agents, antioxidants, UV filters, perfume oils and aromas, dyes or pigments and complete cosmetic or pharmaceutical emulsions are used as are used as component (b3).

10. A process as claimed in at least one of claims 2 to 9, **characterized in that** component (b3) is used in quantities of 0.1 to 25% by weight.

11. A process as claimed in at least one of claims 2 to 10, **characterized in that** the aqueous phase and the oil phase are used in a ratio by weight of 3:1 to 1:3.

12. Use of the polyamide microcapsules claimed in claim 1 for the production of hypochlorite-containing preparations.

13. Use of the polyamide microcapsules claimed in claim 1 for the production of cosmetic preparations.

14. Use of the polyamide microcapsules claimed in claim 1 for finishing textile fabrics.

## Revendications

1. Microcapsules de polyamide d'un diamètre moyen de l'ordre de 0,1 à 5 mm, pouvant être obtenues
(a) en préparant une phase aqueuse dans laquelle sont dissous (a1) des diamines et/ou des triamines et (a2) des tensioactifs,
(b) en préparant une phase huileuse dans laquelle sont dissous (b1) des chlorures d'acide dicarboxylique, (b2) des corps cireux et le cas échéant (b3) des principes actifs, et
(c) en mettant ces deux phases en contact en formant une émulsion H/E, de manière à produire, sur les interfaces, une réaction de polycondensation avec formation de microcapsules de polyamide.

2. Procédé de fabrication de microcapsules de polyamide d'un diamètre moyen de l'ordre de 0,1 à 5 mm, selon lequel
(a) on prépare une phase aqueuse dans laquelle sont dissous (a1) des diamines et/ou des triamines et (a2) des tensioactifs,
(b) on prépare une phase huileuse dans laquelle sont dissous (b1) des chlorures d'acide dicarboxylique, (b2) des corps cireux et le cas échéant (b3) des principes actifs, et
(c) on met ces deux phases en contact en formant une émulsion H/E, de manière à produire sur les interfaces, une réaction de polycondensation avec formation de microcapsules de polyamide.

3. Procédé selon la revendication 2,
**caractérisé en ce qu'**
on utilise de l'hexaméthylènediamine comme diamine (composant a1).

4. Procédé selon les revendications 2 et/ou 3,
**caractérisé en ce que**
comme composant (a2) on utilise des tensioactifs non ioniques choisis dans le groupe formé de :
(a2-1) produits d'addition de 2 à 30 moles d'oxyde d'éthylène et/ou de 0 à 5 moles d'oxyde de propylène sur des alcools gras linéaires comportant de 8 à 22 atomes de C, sur des acides gras comportant de 12 à 22 atomes de C, sur des alkylphénols comportant de 8 à 15 atomes de C dans le groupe alkyle ainsi que des alkylamines comportant de 8 à 22 atomes de carbone dans le radical alkyle,
(a2-2) oligoglycosides d'alkyle et/ou d'alcényle comportant de 8 à 22 atomes de carbone dans le radical alkyle (alcényle) et leur analogues éthoxylés,
(a2-3) produits d'addition de 1 à 15 moles d'oxyde d'éthylène sur de l'huile de ricin et/ou l'huile de ricin durcie,
(a2-4) produits d'addition de 15 à 60 moles d'oxyde d'éthylène sur de l'huile de ricin et/ou l'huile de ricin durcie,
(a2-5) esters partiels de glycérol et/ou de sorbitane et d'acides gras insaturés, linéaires ou saturés, ramifiés comportant de 12 à 22 atomes de carbone et/ou d'acides hydroxycarboxyliques comportant de 3 à 18 atomes de carbone ainsi que leurs produits d'addition avec 1 à 30 moles d'oxyde d'éthylène,
(a2-6) esters partiels de polyglycérol, de polyéthylène glycol, de triméthylolpropane, de pentaérythrite, d'alcools de sucre, de glucosides d'alkyle et de polyglucosides et d'acides gras saturés et/ou insaturés, linéaires ou ramifiés comportant de 12 à 22 atomes de carbone et/ou d'acides hydroxycarboxyliques comportant de 3 à 18 atomes de carbone ainsi que leurs produits d'addition avec 1 à 30 moles d'oxyde d'éthylène,
(a2-7) esters mixtes de pentaérythrite, d'acides gras, d'acide citrique et d'alcool gras et/ou ester mixte d'acides gras comportant de 6 à 22 atomes de carbone, de méthylglucose et de polyols, de préférence de glycérol ou de polyglycérol,
(a2-8) phosphates de mono, di et trialkyle ainsi qu'alkil phosphates de mono, di et/ou tri-PEG- et leurs sels,
(a2-9) alcools de lanoline,
(a2-10) copolymères de polysiloxane-polyalkyle-polyéther ou dérivés correspondants,
(a2-11) copolymères blocs, émulsifiants polymères, polyalkylène glycols ainsi que
(a2-12) carbonate de glycérol.

5. Procédé selon au moins l'une des revendications 2 à 4,
**caractérisé en ce qu'**
on utilise des phases aqueuses qui contiennent les composants (a1) et (a2) chacun en quantités de 0,1 à 5 % en poids.

6. Procédé selon au moins l'une des revendications 2 à 5,
**caractérisé en ce qu'**
on utilise du chlorure d'acide sébacique comme composant (b1).

7. Procédé selon au moins l'une des revendications 2 à 6,
**caractérisé en ce que**
comme composant (b2) on utilise des corps cireux choisis dans le groupe constitué de cires naturelles, de vaseline, de cires de paraffine, de microcires, de cires durcies, de cires de polyalkylène, de cires de polyoléfine, de lécithines et de phospholipides.

8. Procédé selon au moins l'une des revendications 2 à 7,
**caractérisé en ce qu'**
on utilise des phases huileuses qui contiennent les composants (b1) et (b2) chacun en quantités de 0,1 à 5 % en poids.

9. Procédé selon au moins l'une des revendications 2 à 8,
**caractérisé en ce qu'**
comme composant (b3), on utilise des principes actifs choisis dans le groupe constitué d'enzymes, de principes actifs biogènes, d'antioxydants, de filtres UV, d'huiles parfumées et d'arômes, de colorants ou de pigments colorés ainsi que d'émulsions cosmétiques ou pharmaceutiques complètes.

10. Procédé selon au moins l'une quelconque des revendications 2 à 9,
**caractérisé en ce qu'**
on utilise des phases huileuses qui contiennent le composant (b3) en quantités de 0,1 à 25 % en poids.

11. Procédé selon au moins l'une des revendications 2 à 10,
**caractérisé en ce qu'**
on utilise la phase aqueuse et la phase huileuse dans un rapport pondéral de 3:1 à 1:3.

12. Utilisation de microcapsules de polyamide selon la revendication 1, pour l'obtention de préparations contenant de l'hypochlorite.

13. Utilisation de microcapsules de polyamide selon la revendication 1, pour l'obtention de préparations cosmétiques.

14. Utilisation de microcapsules de polyamide selon la revendication 1, pour apprêter des articles textiles plats.
